# EUROPEAN PATENT SPECIFICATION

(11) **EP 3 854 827 B1**
(45) Date of publication and mention of the grant of the patent: **08.11.2023**
(21) Application number: 19862947.9
(22) Date of filing: 09.05.2019
(51) Int. Cl.: C08F 8/42, C08F 8/30, C08F 226/06, H01B 13/00, C12Q 1/26, C12Q 1/00, C07D 231/00, C07F 15/00

(54) **OXIDATION-REDUCTION POLYMER INCLUDING TRANSITION METAL COMPLEX, AND ELECTROCHEMICAL BIOSENSOR USING SAME**
OXIDATIONSREDUKTIONSPOLYMER MIT ÜBERGANGSMETALLKOMPLEX UND ELEKTROCHEMISCHER BIOSENSOR DAMIT
POLYMÈRE D'OXYDO-RÉDUCTION CONTENANT UN COMPLEXE D'UN MÉTAL DE TRANSITION, ET BIOCAPTEUR ÉLECTROCHIMIQUE L'UTILISANT

(30) Priority: 18.09.2018 KR 20180111633
(43) Date of publication of application: 28.07.2021
(73) Proprietor: i-Sens, Inc., Seoul 06646 (KR)
(72) Inventor: KANG, Young Jea, Seoul 01213 (KR); SHIN, Hyunseo, Seoul 04138 (KR); YANG, Bona, Namyangju-si, Gyeonggi-do 12125 (KR); JEONG, In Seok, Seoul 01060 (KR); LEE, Jinseon, Namyangju-si, Gyeonggi-do 12013 (KR); GWAK, Sumin, Incheon 22218 (KR); YANG, Hyunhee, Seoul 07220 (KR)
(74) Representative: Arnold & Siedsma
(86) International application number: PCT/KR2019/006000
(87) International publication number: WO 2020/059997

(56) References cited:
- KR-B1- 101 694 982
- US-A1- 2003 042 137
- US-A1- 2016 296 147
- BEN FELDMAN ET AL: "A Continuous Glucose Sensor Based on Wired Enzyme Technology -Results from a 3-Day Trial in Patients with Type I Diabetes", DIABETIS TECHNOLOGY & THERAPEUTICS, vol. 5, no. 5, 1 October 2003 (2003-10-01) , pages 769-779, XP055730562, MARY ANN LIEBERT, LARCHMONT, NY; US ISSN: 1520-9156, DOI: 10.1089/152091503322526978 Retrieved from the Internet: URL:https://www.liebertpub.com/doi/abs/10. 1089/152091503322526978>
- Ben Feldman, Ronald Brazg, Sherwyn Schwartz, Richard Weinstein: "A Continuous Glucose Sensor Based on Wired Enzyme Technology -Results from a 3-Day Trial in Patients with Type I Diabetes", Diabetes Technology & Therapeutics, vol. 5, no. 5, 1 October 2003 (2003-10-01) , pages 769-779, XP055730562, ISSN: 1520-9156, DOI: 10.1089/152091503322526978
- T J OHara: "Osmium Bipyridyl Redox Polymers Used in Enzyme Electrodes", Platinum Metals Review, vol. 39, no. 2, 1 January 1995 (1995-01-01), pages 54-62, XP002083853,
- Natalia Zabarska, Anne Stumper, Sven Rau: "CuAAC Click Reactions for the Design of Multifunctional Luminescent Ruthenium Complexes", Dalton Transactions, vol. 45, no. 6, 1 January 2016 (2016-01-01), pages 2338-2351, XP055694617, ISSN: 1477-9226, DOI: 10.1039/C5DT04599A

## Description

### [TECHNICAL FIELD]

### CROSS-REFERENCE TO RELATED APPLICATION(S)

This application claims the benefit of Korean Patent Application No. 10-2018-0111633 filed on September 18, 2018 with the Korean Intellectual Property Office.

The present disclosure relates to an oxidation-reduction polymer including a transition metal complex, which can be prepared in a simpler step compared to a conventional method, and can increase the immobilization rate of the transition metal complex and facilitates the introduction of a functional group or a linker, and a method for preparing the same.

### [BACKGROUND ART]

Recently, interest in the development of biosensors is increasing day by day for quantitative and qualitative analysis of target analytes from the medical field to the environment and food fields. In particular, an enzymatic biosensor is a chemical sensor used for selectively detecting and measuring chemical substances contained in a sample by utilizing a biological sensing function in which functional substances of living organisms or organisms such as microorganisms react sensitively with a specific substance, and it has been mainly developed for medical measurement applications such as blood glucose sensors, and is also being actively studied even in applications in the fields of food engineering and environmental measurement.

Periodic measurement of blood glucose is very important in the management of diabetes. Therefore, a wide variety of blood glucose level measuring devices are being prepared so that blood glucose levels can be easily measured using a portable measuring device. The operating principle of such a biosensor is based on an optical method or an electrochemical method. Such an electrochemical biosensor can reduce the influence of oxygen, unlike a biosensor using a conventional optical method, and has the advantage that it can be used without separate pretreatment even if the sample becomes turbid. Therefore, various types of electrochemical biosensors with accuracy and precision are widely used.

Currently commercialized electrochemical blood glucose sensors mainly use enzyme electrodes. More specifically, it has a structure in which a glucose oxidase is immobilized on an electrode capable of converting an electrical signal by a chemical or physical method. These electrochemical blood glucose sensors are based on the principle of measuring the electric current generated by transferring electrons generated by the enzyme oxidation of glucose in analytes such as blood by enzymes to electrodes, thereby measuring the glucose concentration in the analyte. In the case of a biosensor using an enzyme electrode, there is a problem that since the distance from the active center of the enzyme is too far, it is not easy to transfer electrons generated by oxidation of the substrate directedly to the electrode. Therefore, in order to easily carry out such an electron transfer reaction, an oxidation-reduction medium, that is, an electron transfer medium is essentially required. Therefore, it is the type of enzyme used and the characteristics of the electron transfer medium that have the greatest influence on the characteristics of the electrochemical biosensor that measures blood sugar.

In order to block changes in the measured values due to differences in oxygen partial pressure (pO₂) that varies depending on the blood (venous blood, capillary blood, etc.), the development trend of the blood glucose sensor has been changed into the use of GDH, requiring no oxygen in the in the enzymatic reaction, instead of GOX, including oxygen involved in the enzymatic reaction with glucose in the blood. In addition, in the case of electron transfer mediums, organic compounds such as quinone derivatives (phenanthroline quinone, quineonediimine, etc.) and organometallic compounds such as Ru complex (ruthenium hexamine, etc.) or osmium complex, which are excellent in stability according to temperature and humidity, replace the ferricyanide, which is sensitive to stability according to humidity.

A most commonly used electron transfer medium is potassium ferricyanide [K₃Fe(CN)₆]. Because it is inexpensive and has a good reactivity, it can be useful for all sensors using FAD-GOX, PQQ-GDH or FAD-GDH. However, the sensor using the above-mentioned electron transfer medium causes a measurement error due to the interfering substances such as uric acid or gentisic acid present in the blood and is easily deteriorated due to temperature and humidity. Thus, it must be carefully prepared and stored. However, it is difficult to accurately detect low concentration of glucose due to changes in background current after long storage.

Hexaamine ruthenium chloride [Ru(NH₃)₆Cl₃] has higher redox stability than the ferricyanide. Biosensor using this electron transfer medium has advantages in easy manufacturing and storage and has high stability due to small change of background current even when it is stored for a long time. However, it cannot match the reactivity of FAD-GDH, when it is used with FAD-GDH, and thus, it cannot be manufactured as a commercially useful sensor.

Further, in the case of using such a biosensor, obtaining accurate and rapid measurement values with a small amount of samples is a very important problem in terms of maximizing user convenience.

Therefore, the development of a new electron transport medium capable of achieving the reduction of the disadvantages and measurement time of the conventional electron transport medium is still required.

Meanwhile, a continuous glucose monitoring (CGM) system is used to continuously monitor blood glucose levels and manage diseases such as diabetes, and existing enzyme sensors that collect blood from the fingertips induce a considerable pain due to a needle during blood collection and thus limits the measurement frequency and cannot be used for such CGM. In order to solve these problems, an improved version of a continuous blood glucose monitoring enzyme sensor that can adhere to the body and thus minimize invasion has recently been developed. In the case of such as continuous blood glucose monitoring enzyme sensor, since part of the sensor enters the human body, the electron transfer medium containing transition metals is absorbed into the human body and fixed with a polymer backbone such as polyvinylpyridine or polyvinylimidazole so that toxicity and side effects do not occur, whereby it is intended to prevent problems caused by the loss of the electron transfer medium in the human body.

In this way, in the case of the oxidation-reduction polymer to which the electron transfer medium is connected, conventionally, coupling reaction using N-hydroxysuccinimide (NHS), an active ester, was mainly used in order to efficiently fix the transition metal complex to the main polymer backbone. However, in the case of such an existing synthesis method, it has to go through very complicated steps until it is synthesized into the final oxidation-reduction polymer. In addition, when the coupling reaction using NHS proceeds, the immobilization efficiency of the transition metal complex due to hydrolysis is not actually high, and there was a problem that it was difficult to introduce other types of linkers or functional groups into the main backbone of the polymer.

US2016/296147A1 and BEN FELDMAN et al. in "A Continuous Glucose Sensor Based on Wired Enzyme Technology -Results from a 3-Day Trial in Patients with Type I Diabetes",DIABETIS TECHNOLOGY & THERAPEUTICS, vol. 5, no. 5, 1 October 2003 (2003-10-01), pages 769-779, disclose redox polymers that are used to make biosensors.

Therefore, there is an increasing demand for the development of oxidation-reduction polymers for a biosensor that can solve these problems.

### [DETAILED DESCRIPTION OF THE INVENTION]

### [Technical Problem]

The present disclosure has been devised to solve the above-mentioned problems, and thus, it is an object of the present disclosure to provide an oxidation-reduction polymer including a transition metal complex, which can be prepared in a simpler step compared to a conventional method, can increase the immobilization rate of the transition metal complex and facilitates the introduction of a functional group or a linker, and a method for preparing the same.

It is another object of the present disclosure to provide an electrochemical biosensor including an oxidation-reduction polymer including a transition metal complex.

### [Technical Solution]

In order to achieve the above objects, the present disclosure provides an oxidation-reduction polymer including a transition metal complex, which can be easily synthesized by utilizing an azide-alkyne cycloaddition reaction using copper catalyst and heat, and a thiol-ene click reaction using light, exhibits an improved immobilization rate of the transition metal complex, and facilitates the introduction of a functional group or a linker, and an electrochemical biosensor, such as a blood glucose sensor.

The oxidation-reduction polymer including the transition metal complex according to the present disclosure includes a transition metal complex including polymer backbones such as poly(vinylpyridine) (PVP) or poly(vinylimidazole) (PVI), and transition metals such as osmium, ruthenium, iridium, rhodium, iron, or cobalt, and a ligand thereof, and a linker structure connecting the polymer backbone and the transition metal complex. Specifically, the oxidation-reduction polymer has the structure of the following Chemical Formulas 1 to 4: 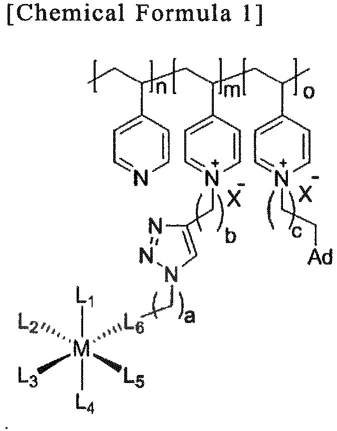 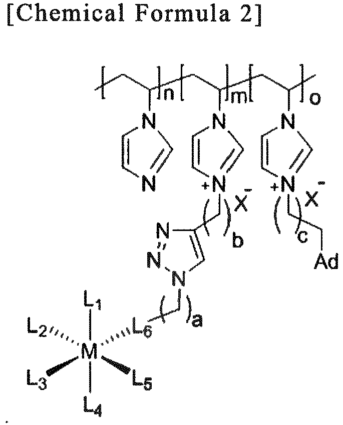 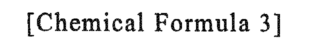 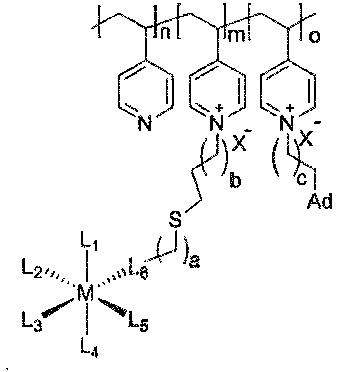 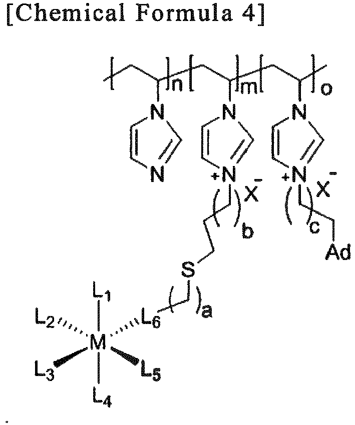 wherein,
M is a transition metal selected from the group consisting of Os, Rh, Ru, Ir, Fe and Co;
L₁ and L₂ are combined with each other to form a bidentate ligand selected from the following Chemical Formulas 5 to 7;
L₃ and L₄ are combined with each other to form a bidentate ligand selected from the following Chemical Formulas 5 to 7;
L₅ and L₆ are each combined with each other to form a bidentate ligand selected from the following Chemical Formulas 5 to 7; 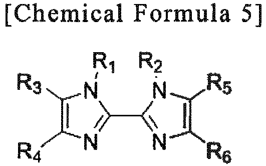 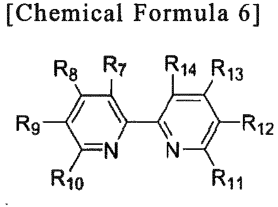 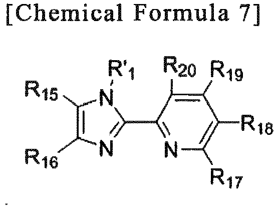
the R₁, R₂ and R'₁ are each independently selected from the group consisting of a substituted or unsubstituted alkyl group having 1 to 10 carbon atoms, a substituted or unsubstituted alcohol group having 1 to 20 carbon atoms, a substituted or unsubstituted alkylhalogen group having 1 to 20 carbon atoms, a substituted or unsubstituted thiol group having 1 to 20 carbon atoms, a substituted or unsubstituted alkyl azide group having 3 to 20 carbon atoms, a substituted or unsubstituted aryl azide group having 7 to 30 carbon atoms, a substituted or unsubstituted alkenyl group having 2 to 40 carbon atoms, a substituted or unsubstituted alkynyl group having 2 to 40 carbon atoms, a cyano group, a halogen group, deuterium and hydrogen,
the R₃ to R₂₀ are each independently selected from the group consisting of a substituted or unsubstituted alkyl group having 1 to 10 carbon atoms, a substituted or unsubstituted cycloalkyl group having 3 to 40 carbon atoms, a substituted or unsubstituted aryl group having 6 to 50 carbon atoms, a substituted or unsubstituted heteroaryl group having 3 to 50 carbon atoms, a substituted or unsubstituted alkoxy group having 1 to 20 carbon atoms, a substituted or unsubstituted alcohol group having 1 to 20 carbon atoms, a substituted or unsubstituted alkylhalogen group having 1 to 20 carbon atoms, a substituted or unsubstituted thiol group having 1 to 20 carbon atoms, a substituted or unsubstituted alkyl azide group having 3 to 20 carbon atoms, a substituted or unsubstituted aryl azide group having 7 to 30 carbon atoms, a substituted or unsubstituted aryloxy group having 6 to 30 carbon atoms, a substituted or unsubstituted alkylamino group having 1 to 20 carbon atoms, a substituted or unsubstituted arylamino group having 6 to 30 carbon atoms, a substituted or unsubstituted alkylsilyl group having 1 to 20 carbon atoms, a substituted or unsubstituted arylsilyl group having 6 to 30 carbon atoms, a substituted or unsubstituted arylalkylamino group having 1 to 50 carbon atoms, a substituted or unsubstituted alkenyl group having 2 to 40 carbon atoms, a substituted or unsubstituted alkynyl group having 2 to 40 carbon atoms, a cyano group, a halogen group, deuterium and hydrogen;
the Ad is selected from the group consisting of a primary and secondary amine group, an ammonium group, a halogen group, an epoxy group, an azide group, an acrylate group, an alkenyl group, an alkynyl group, a thiol group, isocyanate, an alcohol group, and a silane group;
the X is a counter ion;
the a is an integer of 1 to 15;
the b is an integer of 1 to 15;
the c is an integer of 1 to 15;
the m is an integer of 10 to 600;
the n is an integer of 10 to 600; and
the o is an integer of 0 to 600.

The oxidation-reduction polymer including the transition metal complex provided in the present disclosure has a unique linker structure, and so it has an advantage that the number of synthesis steps is reduced compared to the existing case and so the production can be carried out in a simple step, the immobilization rate of the transition metal complex is increased, and the introduction of a functional group or a linker is easy. In addition, it is preferable that the oxidation-reduction polymer including the transition metal complex provided in the present disclosure has three kinds of bidentate ligands. Therefore, the electrochemical biosensor including such an oxidation-reduction polymer, preferably a continuous blood glucose monitoring sensor, and has advantages of being prepared economically, significantly reducing toxicity and side effects due to transition metals, and having a high yield during production.

An embodiment of the present disclosure relates to an electrochemical biosensor, which is prepared by applying an enzyme capable of subjecting a liquid biological sample to an oxidation-reduction with the oxidation-reduction polymer having Chemical Formulas 1 to 4 onto a substrate having at least two electrodes, and then drying the same. Examples of the electrode may be a working electrode and a counter electrode. For example, enzymes and transition metal polymers can be applied to or placed in close proximity to the working electrode.

In specific embodiments, although a biosensor for measuring glucose level is presented as an example of the applicable electrochemical biosensor, but the present disclosure can be applied to a biosensor for quantification of various substances such as cholesterol, lactate, creatinine, hydrogen peroxide, alcohol, amino acid, and glutamate by varying the types of enzymes contained in the reagent composition of the present disclosure.

Hereinafter, the present disclosure will be described in more detail.

Preferably, in the oxidation-reduction polymer of Chemical Formulas 1 to 4 according to the present disclosure, the counterion of X may be selected from the group consisting of an anion, for example, a halide which may be selected from the group consisting of F, Cl, Br and I, sulfate, phosphate, hexafluorophosphate, tetrafluoroborate, or a cation (preferably monovalent cations), for example lithium, sodium, potassium, tetraalkylammonium and ammonium. More preferably, X may be chloride.

Preferably, the a may be an integer of 2 to 10.

Preferably, the b may be an integer of 2 to 10.

Preferably, the c may be an integer of 2 to 10.

Preferably, the m may be an integer of 15 to 550.

Preferably, the n may be an integer of 15 to 550.

Preferably, the o may be an integer of 0 to 300.

Preferably, the oxidation-reduction polymer medium according to the present disclosure may have a structure represented by the following Chemical Formula 8 or 9, without being limited thereto.

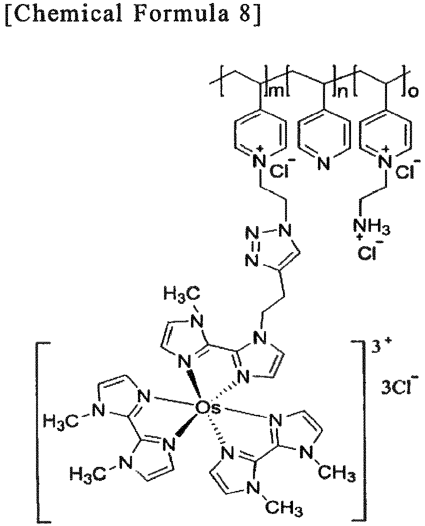

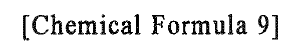

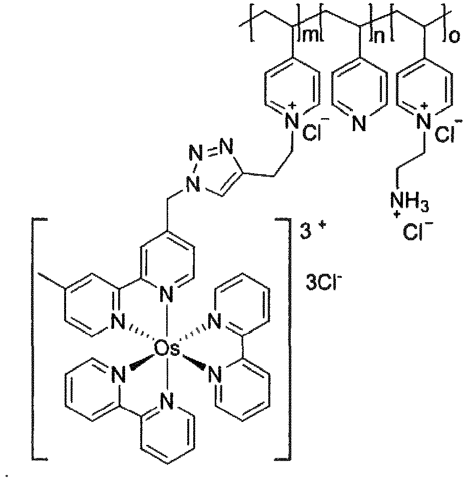

The transition metal complex in the oxidation-reduction polymer having a structure selected from Chemical Formulas 1 to 4 according to the present disclosure may include, specifically, an osmium complex, for example, a trivalent osmium complex and a divalent osmium complex, and the osmium complex may preferably be an oxidized compound (trivalent Os compound). The oxidizing agent used in the oxidation treatment of the present disclosure is not particularly limited, and specific examples may be one or more selected from the group consisting of NaOCl, H₂O₂, O₂, O₃, PbO₂, MnO₂, KMnO₄, ClO₂, F₂, Cl₂, H₂CrO₄, N₂O, Ag₂O, OsO₄, H₂S₂O₈, ceric ammonium nitrate (CAN), pyridinium chlorochromate, and 2,2'-dipyridyldisulfide. In addition, in the case of a mixture containing a compound in an oxidized state and a reduced state as a transition metal complex, the transition metal complex in an oxidized state or the chloride compound of a transition metal complex in an oxidized state can be provided by the oxidation treatment.

The transition metal complex according to the present disclosure may be in the form of a salt having suitable counter ions and ions. The salt compound are more preferable because they have high solubility in water or other aqueous solutions or organic solvents. When it consists of small counter anions such as F-, Cl- and Br- in the chloride compound, it tends to be dissolved well in water or various aqueous solutions, and groups consisting of large counter anions such as I-, hexafluorophosphate (PF₆⁻) and tetrafluoroborate (BF₄⁻) tend to dissolve well in organic solvents. Thus, the counter anion may be one or more salt compounds selected from the group consisting of a halide, which may be selected from the group consisting of F, Cl, Br and I, hexafluorophosphate and tetrafluoroborate, and the counter cation may be one or more salt compounds selected from the group consisting of Li salt, Na salt, K salt, Rb salt, Cs salt, Fr salt, tetraalkylammonium and ammonium, without being limited thereto.

In a specific embodiment, the oxidation-reduction polymer according to the present disclosure may be synthesized by reacting a polymer backbone and a transition metal complex by a click reaction. Specifically, the compound according to Chemical Formula 1 of the oxidation-reduction polymer according to the present disclosure may be prepared by the following azide-alkyne Huisgen cycloaddition reaction, which is represented by Reaction Scheme 1, without being limited thereto.

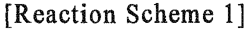

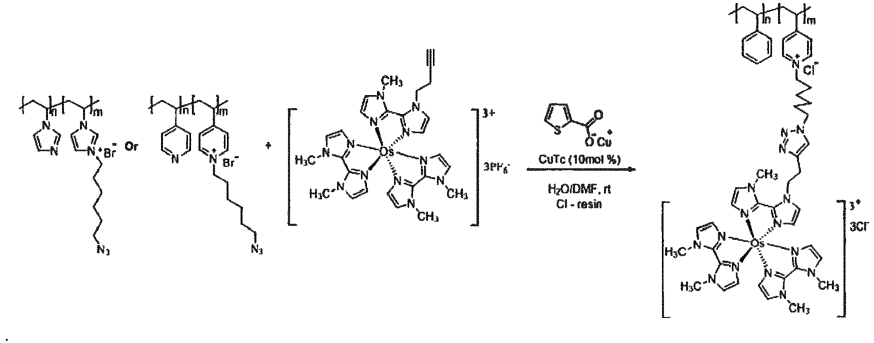

Further, the compound of Chemical Formula 2 may be prepared by the following thiol-ene reaction, which may be represented by Reaction Scheme 2, without being limited thereto.

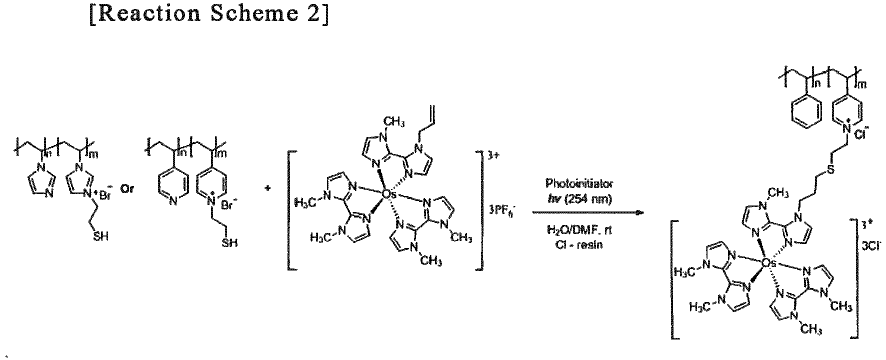

Preferably, as a starting material for preparing an oxidation-reduction polymer according to Reaction Scheme 1 or 2, polyvinylpyridine or polyvinylimidazole can be functionalized to give a polyvinylpyridine or polyvinylimidazole precursor. The functionalized polyvinylpyridine or polyvinylimidazole precursor may have a structure represented by the following Chemical Formula 10 or 11.

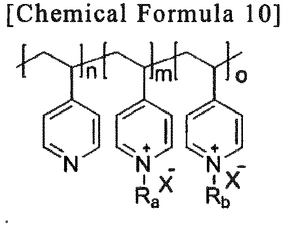

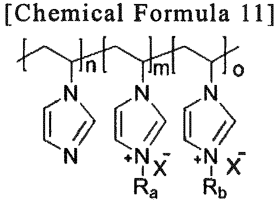

In the structure of the polymer precursor, Rₐ and R_{b} are selected from the group consisting of a substituted or unsubstituted alkyl group having 1 to 10 carbon atoms, a substituted or unsubstituted alcohol group having 1 to 10 carbon atoms, a substituted or unsubstituted alkylhalogen group having 1 to 20 carbon atoms, a substituted or unsubstituted thiol group having 1 to 20 carbon atoms, a substituted or unsubstituted alkyl azide group having 3 to 20 carbon atoms, a substituted or unsubstituted aryl azide group having 7 to 30 carbon atoms, a substituted or unsubstituted alkenyl group having 2 to 40 carbon atoms, and a substituted or unsubstituted alkynyl group having 2 to 40 carbon atoms.

The functionalized polyvinylpyridine or polyvinylimidazole precursor can be synthesized, for example, as shown in the following Reaction Scheme 3.

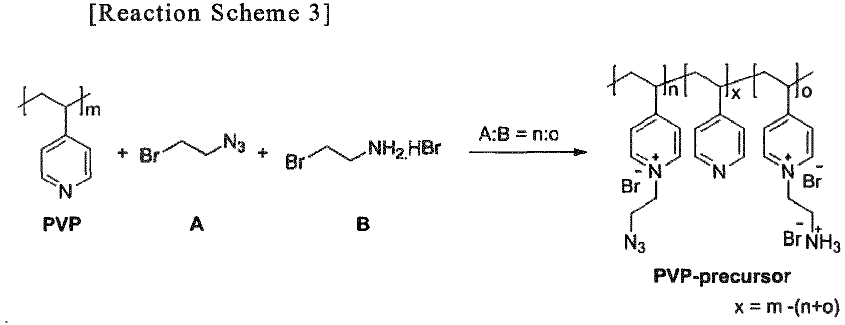

Preferably, as a starting material for preparing an oxidation-reduction polymer according to Reaction Scheme 1 or 2, the transition metal complex may be functionalized. The functionalized transition metal complex can be synthesized, for example, as shown in the Reaction Schemes 4 and 5.

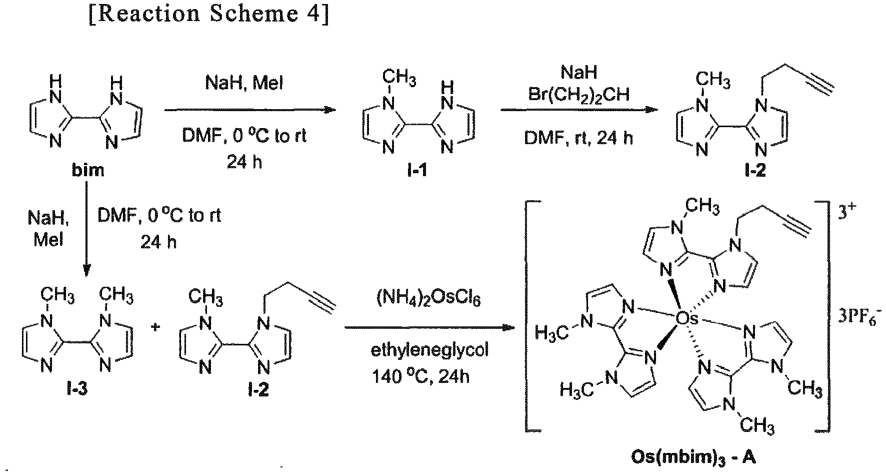

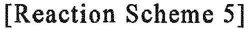

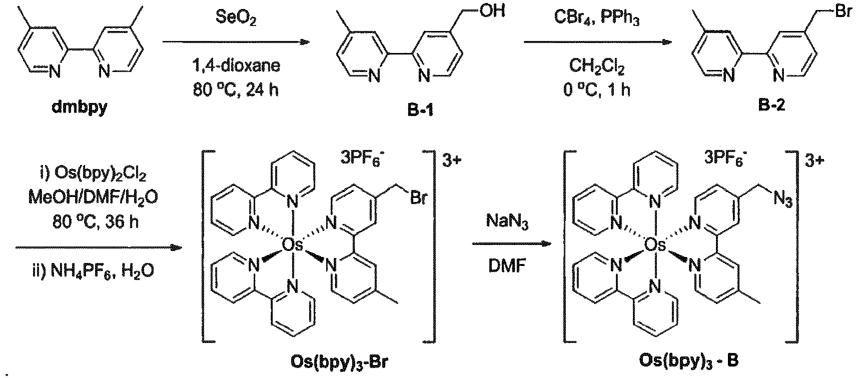

Therefore, in a further embodiment, the present disclosure relates to a method for preparing an oxidation-reduction polymer of Chemical Formula 1 or 2 comprising the steps of:
(i) functionalizing polyvinylpyridine or polyimidazole to produce a polyvinylpyridine precursor or a polyimidazole precursor;
(ii) functionalizing the transition metal complex; and
(iii) reacting the polyvinylpyridine precursor or polyimidazole precursor produced in step (i) and the functionalized transition metal complex produced in step (ii) by a click reaction to prepare the oxidation-reduction polymer of Chemical Formula 1 or 2.

Specific embodiments of each step are as described above.

The transition metal complex of the oxidation-reduction polymer according to the present disclosure not only enables accurate, reproducible, rapid and continuous analysis of the target material, but also can be produced easily and economically in high yields, and has the advantage that the problems of toxicity and side effects that can be caused by the outflow of transition metals are significantly low.

The oxidation-reduction polymer according to the present disclosure may be applied or laminated on the working electrode or may be located around the working electrode (for example, a structure surrounding the electrode in a solution) to transfer electrons between the working electrode and the substance to be analyzed via an enzyme. The oxidation-reduction polymer can form non-filterable coatings on the working electrode within the electrochemical biosensor.

A further embodiment of the present disclosure relates to a composition for an electrochemical biosensor comprising an enzyme capable of subjecting a liquid biological sample to an oxidation-reduction reaction, and the oxidation-reduction polymer.

The oxidoreductase is a generic term for an enzyme that catalyzes the redox reaction in a living organism. In the case of a target substance to be measured in the present disclosure, such as a biosensor, the oxidoreductase refers to an enzyme that is reduced by reacting with a target substance to be measured. The enzyme reduced in this way reacts with the electron transport medium and generate signal such as current change, and the metabolite is quantified by measuring the signal such as the current change occurring at this time. The oxidoreductase usable in the present disclosure may be at least one selected from the group consisting of various dehydrogenase, oxidase, esterase. Depending on the redox reaction or detection target material, an enzyme using the substrate as the target material may be selected and used among enzymes belonging to the enzyme group.

More specifically, the oxidoreductase may be one or more selected from the group consisting of glucose dehydrogenase, glutamate dehydrogenase, glucose oxidase, cholesterol oxidase, cholesterol esterase, lactate oxidase, ascorbic acid oxidase, alcohol oxidase, alcohol dehydrogenase, bilirubin oxidase.

Meanwhile, the oxidoreductase can also include a cofactor that plays a role of storing hydrogen deprived by the oxidoreductase from the target substance (e.g., metabolite) to be measured. For example, the cofactor may be one or more selected from the group consisting of flavin adenine dinucleotide (FAD), nicotinamide adenine dinucleotide (NAD), pyrroloquinoline quinone (PQQ).

For examples, when measuring the blood glucose concentration, glucose dehydrogenase (GDH) may be used as an oxidoreductase, and may include flavin adenine dinucleotide-glucose dehydrogenase (FAD-GDH) containing FAD as the cofactor and/or nicotinamide adenine dinucleotide-glucose dehydrogenase containing FAD-GDH as the cofactor.

In an embodiment, the available oxidoreductase may be at least one selected from the group consisting of FAD-GDH (e.g., EC 1.1.99.10 etc.), NAD-GDH (e.g., EC 1.1.1.47 etc.), PQQ-GDH (e.g., EC1.1.5.2 etc.), glutamate dehydrogenase (e.g., EC 1.4.1.2 etc.), glucose oxidase (e.g., EC 1.1.3.4 etc.), cholesterol oxidase (e.g., EC 1.1.3.6 etc.), cholesterol esterase (e.g., EC 3.1.1.13 etc.), lactate oxidase (e.g., EC 1.1.3.2 etc.), ascorbic acid oxidase (e.g., EC 1.10.3.3 etc.), alcohol oxidase (e.g., EC 1.1.3.13 etc.), alcohol dehydrogenase (e.g., EC 1.1.1.1 etc.), bilirubin oxidase (e.g., EC 1.3.3.5 etc.).

The composition according to the present disclosure may contain 20 to 700 parts by weight, for example, 60 to 700 parts by weight or 30 to 340 parts by weight of an oxidation-reduction polymer, based on 100 parts by weight of the oxidoreductase. The content of the oxidation-reduction polymer may be appropriately adjusted in accordance with the activity of the redox enzyme.

In addition, the composition according to the present disclosure may further include a crosslinking agent.

Meanwhile, the composition according to the present disclosure may further include one or more additives selected from the group consisting of surfactants, water-soluble polymers, quaternary ammonium salts, fatty acids, thickeners, etc., for the role of a dispersant during reagent dissolution, an adhesive during reagent production, a stabilizer for long-term storage.

The surfactant may play a role in allowing the composition to spread evenly over the electrodes and be dispensed with a uniform thickness when dispensing the reagents. As the surfactant, at least one selected from the group consisting of Triton X-100, sodium dodecyl sulfate, perfluorooctane sulfonate, sodium stearate, etc. may be used. In order to properly perform the role of spreading the reagent uniformly on the electrodes and dispensing the reagent with uniform thickness when dispensing the reagent, the reagent composition according to the present disclosure may contain the surfactant in an amount of 3 to 25 parts by weight, for example 10 to 25 parts by weight, based on 100 parts by weight of the oxidoreductase. For example, when using an oxidoreductase with an activity of 700 U/mg, the reagent composition may contain 10 to 25 parts by weight of a surfactant based on 100 parts by weight of the oxidoreductase. When the activity of the oxidoreductase is higher than that, the content of the surfactant can be adjusted to lower level.

The water-soluble polymer may serve to stabilize and disperse enzymes as a polymer support for the reagent composition. The water-soluble polymers used herein may include at least one selected from the group consisting of polyvinyl pyrrolidone (PVP), polyvinyl alcohol (PVA), polyfluoro sulfonate, hydroxyethyl cellulose (HEC), and hydroxypropyl cellulose (HPC), carboxy methyl cellulose (CMC), cellulose acetate, polyamide. The reagent composition according to the present disclosure may contain the water-soluble polymer in an amount of 10 to 70 parts by weight, for example 30 to 70 parts by weight based on 100 parts by weight of the oxidoreductase, in order to sufficiently and appropriately exhibit the role of assisting the stabilization and dispersing of oxidoreductase. For example, when using an oxidoreductase having an activity of 700 U/mg, the composition may contain 30 to 70 parts by weight of a water-soluble polymer based on 100 parts by weight of the oxidoreductase. If the activity of the oxidoreductase is higher than that, the content of the water-soluble polymer can be adjusted to lower level.

The water-soluble polymer may have a weight average molecular weight of about 2,500 to 3,000,000, for example, about 5,000 to 1,000,000, in order to effectively assist the stabilization and dispersion of a support and an enzyme.

The thickener serves to firmly adhere the reagent to the electrode. As the thickener, at least one selected from the group consisting of natrosol, diethylaminoethyl-dextran hydrochloride, may be used. The electrochemical sensor according to the present disclosure may contain the thickener in an amount of 10 to 90 parts by weight, for example, 30 to 90 parts by weight, based on 100 parts by weight of the oxidoreductase, in order to ensure that the oxidation-reduction polymer according to the present disclosure is firmly attached to the electrode. For example, when using an oxidoreductase having an activity of 700 U/mg, it may contain 30 to 90 parts by weight of a thickener based on 100 parts by weight of the oxidoreductase, and when the activity of the oxidoreductase is higher than that, the content of the thickener can be adjusted to lower level.

In a further embodiment, the present disclosure provides an electrochemical biosensor including the oxidation-reduction polymer.

Specifically, the type of the electrochemical biosensor is not limited, but a continuous blood glucose monitoring sensor can be preferably used.

In the configuration of such a continuous blood glucose monitoring sensor, the present disclosure may include, for example, an electrode, an insulator, a substrate, a sensing layer, a diffusion layer, a protection layer, which include the oxidation-reduction polymer and the oxidoreductase. In the case of an electrode, it may include two types of electrodes such as a working electrode and a counter electrode, and it may also include three types of electrodes such as a working electrode, a counter electrode, and a reference electrode. In one embodiment, the biosensor according to the present disclosure may be an electrochemical biosensor prepared by coating a reagent composition containing an electron transfer medium and an enzyme capable of subjecting a liquid biological sample to an oxidization-reduction, onto a substrate having at least two, preferably two or three electrodes, and then drying it. For example, there is provided a planar electrochemical biosensor, characterized in that in the electrochemical biosensor, an working electrode and a counter electrode are provided on opposite surfaces of a substrate, and a sensing layer containing the oxidation-reduction polymer according to the present disclosure is stacked on the working electrode, and an insulator, a diffusion layer and a protective film are sequentially stacked on both sides of a substrate having an working electrode and a counter electrode.

In a specific embodiment, the substrate may be made of one or more materials selected from the group consisting of polyethylene terephthalate (PET), polycarbonate (PC), and polyimide (PI).

Further, as the working electrode, a carbon, gold, platinum, silver or silver/silver chloride electrode may be used.

Further, in the case of an electrochemical biosensor having a 2-electrode, since the counter electrode performs up to the role of a reference electrode, gold, platinum, silver or silver/silver chloride electrodes can be used as the counter electrode. In the case of a 3-electrode electrochemical biosensor including up to the reference electrode, a gold, platinum, silver, or silver/silver chloride electrode may be used as the reference electrode, and a carbon electrode may be used as the counter electrode.

Nafion, cellulose acetate, silicone rubber can be used as the diffusion layer, and silicone rubber, polyurethane, polyurethane-based copolymer, can be used as the protective film, without being limited thereto.

As a non-limiting example, in the case of the 2-electrode, silver chloride or silver may be used because the counter electrode performs up to the role of the reference electrode, and in the case of the 3-electrode, silver chloride or silver may be used as the reference electrode, and a carbon electrode may be used as the counter electrode.

### [ADVANTAGEOUS EFFECTS]

The oxidation-reduction polymer according to the present disclosure has a small number of steps during production, and so is economical, has an increased immobilization rate of a transition metal complex, and facilitates the introduction of a functional group or a linker, whereby biosensors to which this is applied have the advantages of being simple, quick to detect and economical.

### [BRIEF DESCRIPTION OF THE DRAWINGS]

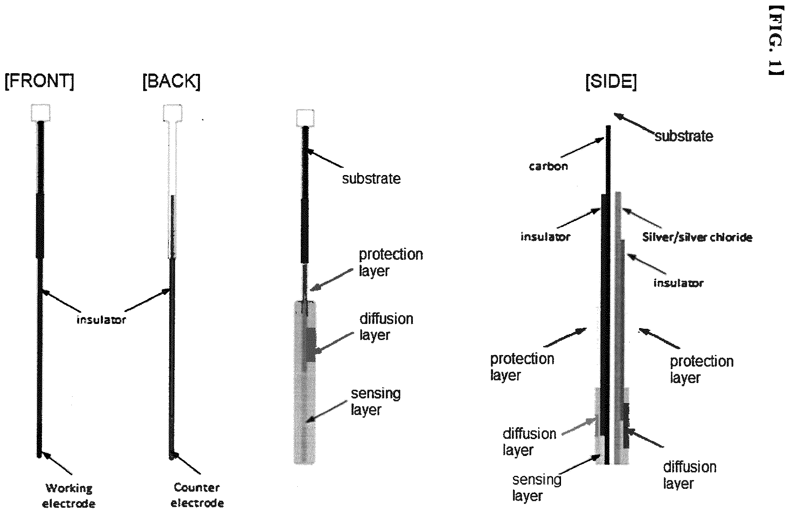

FIG. 1 is a diagram showing the structure of a biosensor according to an embodiment of the present disclosure.
FIG. 2 is a graph showing the results of measuring a cyclic voltammetry curve using the compound of Chemical Formula 8 which is the oxidation-reducing polymer for the electron transport medium according to the present disclosure, and a single Os complex.

### [DETAILED DESCRIPTION OF THE EMBODIMENTS]

Hereinafter, the present disclosure will be described in more detail.

The present disclosure will be described in more detail by way of examples. However, the present disclosure is for illustrative purposes only and the scope of the present disclosure is not limited thereto.

### Preparation Example 1: Synthesis of an oxidation-reduction polymer compound represented by [Chemical Formula 8]

### 1-1. Synthesis of 2,2'-biimidazole

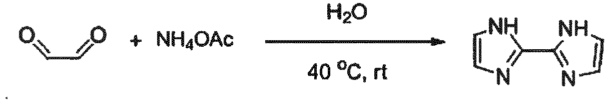

79 mL (0.69 mol) of 40% glyoxal aqueous solution was added to a 500 mL three-neck round-bottom flask, cooled to 0°C, and then 370 mL (2.76 mol) of ammonium acetate was slowly added dropwise through a dropping funnel for 3 hours, while paying attention to temperature rise (less than 30°C). After completion of the dropwise addition, the mixture was stirred overnight at 45-50 °C, and then cooled to room temperature. The resulting solid was filtered, then dissolved in ethyl glycol, and purified by a hot-filter. Finally, 2,2'-biimidazole was obtained. (10.1 g, yield: 33%)

### 1-2. Synthesis of N-methyl-2,2'-biimidazole

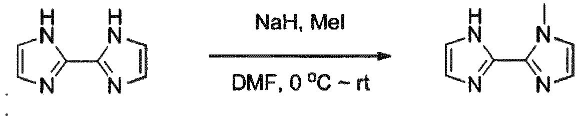

2 g (15 mmol) of 2,2'-biimidazole was added to a 250 mL three-neck round-bottom flask, dissolved in 60 mL of anhydrous dimethylformamide, and then cooled to 0 °C. 0.6 g (15 mmol) of sodium hydride was added little by little, while paying attention to temperature rise. The mixture was stirred at 0 °C for 1 hour, and then 1 mL (15 mmol) of iodomethane was slowly added dropwise through a syringe pump. After completion of the dropwise addition, the mixture was stirred at room temperature for 12 hours. 100 mL of ethyl acetate was added to the final reaction solution, and the resulting sodium iodide was removed by filtration. The filtrate was concentrated under reduced pressure to remove the solvent, and then the remaining solid was purified by column chromatography using ethyl acetate and hexane as developing solvents. Finally, *N*-methyl-2,2'-biimidazole was obtained. (0.8 g, yield: 37%)

### 1-3. Synthesis of N,N'-dimethyl-2,2'-biimidazole

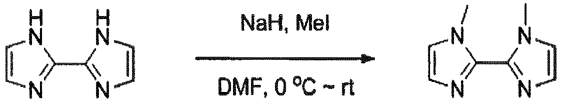

5 g (37 mmol) of 2,2'-biimidazole was added to a 500 mL three-neck round-bottom flask, dissolved in 60 mL of anhydrous dimethylformamide, and then cooled to 0 °C. 3 g (40 mmol) of sodium hydride was added little by little, while paying attention to temperature rise. The mixture was stirred at 0°C for 1 hour, and then 2.5 mL (40 mmol) of iodomethane was slowly added dropwise through a syringe pump. After completion of the dropwise addition, the mixture was stirred at room temperature for 24 hours. Water was added to the final reaction solution, extracted with ethyl acetate (200 mL X 3), and then the organic layer was collected and dried over magnesium sulfate. The organic layer was concentrated under reduced pressure to remove the solvent, and then purified by column chromatography using ethyl acetate and hexane as developing solvents. Finally, *N,N*'-dimethyl-2,2'-biimidazole was obtained. (5.1 g, yield: 84%)

### 1-4. Synthesis of N-butynyl-N'-methyl-2,2'-biimidazole

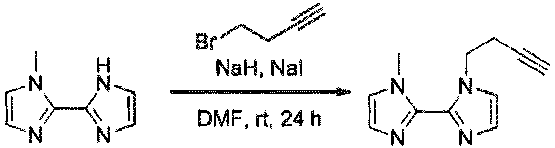

1.5 g (10 mmol) of N-methyl-2,2'-biimidazole was added to a 100 mL three-neck round-bottom flask, dissolved in 30 mL of anhydrous dimethylformamide under nitrogen, and then sodium hydride 0.5 g (13 mmol) was added thereto. The mixture was stirred at room temperature for 1 hour, and then 1.7 g (13 mmol) of 4-bromo-1-butyne and 1.5 g (10 mmol) of sodium iodide were added thereto. The reaction solution was heated to 80 °C under nitrogen and stirred for 24 hours. The final reaction solution was cooled to room temperature, extracted with water (100 mL) and ethyl acetate (200 mL X 3), and then the organic layer was collected and dried over magnesium sulfate. The organic layer was concentrated under reduced pressure to remove the solvent, and purified by column chromatography using ethyl acetate and hexane as developing solvents. Finally, N-butynyl-N'-methyl-2,2'-biimidazole was obtained. (1.5 g, yield: 74%)

### 1-5. Synthesis of [Osmium (III) (N,N'-dimethyl-2,2'-biimidazole)₂ (N-butynyl-N'-methyl-2,2'-biimidazole)](hexafluorophosphine)₃

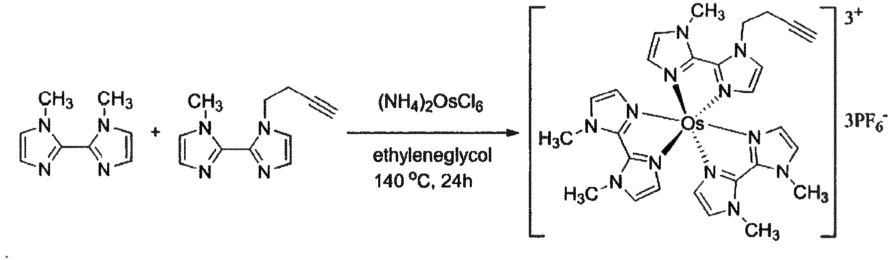

A 100 mL three-neck round-bottom flask was equipped with a reflux condenser, a gas inlet and a thermometer, and 2 g (13 mmol) of *N,N'-*dimethyl-2,2'-biimidazole, 3 g (6.5 mmol) of ammonium hexachloro osmate (IV) and 50 mL of ethylene glycol were stirred under nitrogen at 140 °C for 24 hours. 1.3 g (6.5 mmol) of *N*-butynyl-*N*'-methyl-2,2'-biimidazole was dissolved in 10 mL of ethylene glycol, and then added to the reaction mixture using a syringe. The mixture was again stirred at 140 °C under nitrogen for 24 hours. After completion of the reaction, the reaction mixture was cooled to room temperature, and the resulting red residue was removed by filtration. The filtrate was diluted with 300 mL of water, and then AG1X4 chloride resin was added and stirred for 24 hours to sufficiently oxidize in air. The solution is added dropwise to an aqueous ammonium hexafluorophosphine solution to obtain a precipitate of the ion-exchanged metal complex. The resulting solid was filtered, washed several times with water, and then dried in a vacuum oven to obtain the final compound osmium (III) complex. (5 g, yield: 67%)

### 1-6. Synthesis of poly(4-(2-azidoethyl)pyridinium)-co-(4-(2-aminoethyl)pyridinium)-co-4-vinylpyridine)

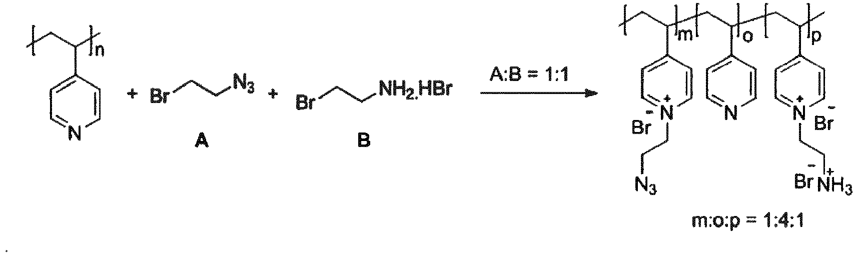

A 250 mL three-neck round-bottom flask was equipped with a reflux condenser, a gas inlet and a thermometer, and 20 g of poly(4-vinylpyridine): number average molecular weight: -160,000 g/mol) was dissolved in 150 mL of dimethylformamide. 4.5 g (30 mmol) of 1-azido-2-bromoethane and 6.0 g (30 mmol) of 2-bromoethylamine were added to this solution. The solution was stirred at 90 °C for 24 hours using a mechanical stirrer. After completion of the reaction, the reaction mixture was cooled to room temperature and poured into ethyl acetate solution to form a precipitate. The solvent was drained off, and the resulting solid was dissolved again in 300 mL of methanol, concentrated under reduced pressure (150 mL), and a precipitate was again formed in diethyl ether. The resulting solid was dried in a vacuum oven to obtain a polyvinylpyridine precursor. (27 g, yield: 90%)

### 1-7. Synthesis of Oxidation-Reduction Polymer 1 [Chemical Formula 8]

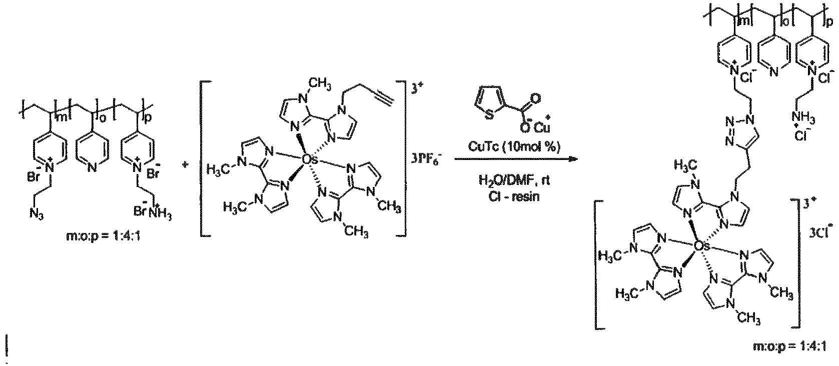

0.5 g of poly(4-(2-azidoethyl)pyridinium)-co-(4-(2-aminoethyl) pyridinium)-co-4-vinylpyridine) was added to a 50 mL culture tube and dissolved in 10 mL of distilled water. Then, 0.8 g of [Osmium(III)(*N*,*N*'-dimethyl-2,2'-biimidazole)₂(*N*-butynyl-*N*'-methyl-2,2'-biimidazole)](hexafluorophosphine)₃ dissolved in 5 mL of dimethylformamide was added thereto. 25 mg of a copper (I) catalyst (CuTc: Copper(I)thiophene carboxylate) was added to the reaction mixture and stirred at room temperature for 12 hours. After completion of the reaction, the reaction mixture was poured into ethyl acetate solution to form a precipitate. The solvent was drained off and the resulting solid was dissolved again in 50 mL of acetonitrile, and AG1X4 chloride resin and water (150 mL) were added and stirred for 24 hours. The polymer solution is concentrated under reduced pressure (50 mL), and then dialyzed to remove substances of low molecular weight (10,000 g/mol or less). The dialyzed polymer solution was lyophilized to obtain a final oxidation-reduction polymer 1. (0.7 g)

### Experimental Example: Confirmation of the electrochemical properties of the oxidation-reduction polymer [Formula 8] and Os complex for an electron transfer medium according to the present disclosure using cyclic voltammetry

In order to confirm the performance of the oxidation-reduction polymer including the Os complex according to the present disclosure as an electron transfer medium, electrochemical properties were measured using the cyclic voltammetry method according to the following experimental method.

### Experimental method

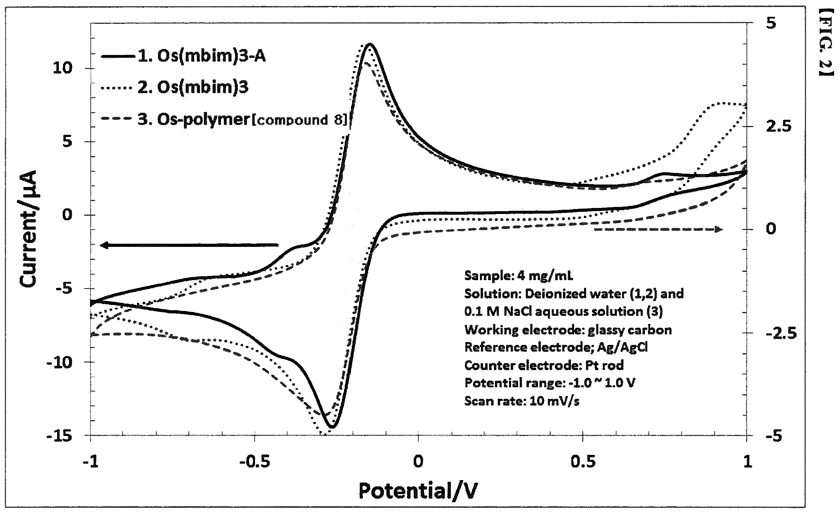

① Two kinds of osmium complexes of the following Chemical Formulas 12 and 13 (Os(mbim)₃, Os(mbim)₃-A), and 20 mg of each of the osmium-polymers of Chemical Formula 8 according to the present disclosure were dissolved in deionized water and 5 mL of 0.1M sodium chloride solution.
② Degassed with argon for 10 minutes to remove oxygen in the solution.
③ The working electrode, the reference electrode, and the counter electrode were connected to the oxygen-degassed solution, and changes in the electrical signal due to changes in the voltage were measured under argon.
(3) The results of the experiment are shown in FIG. 2 and Table 1, respectively.

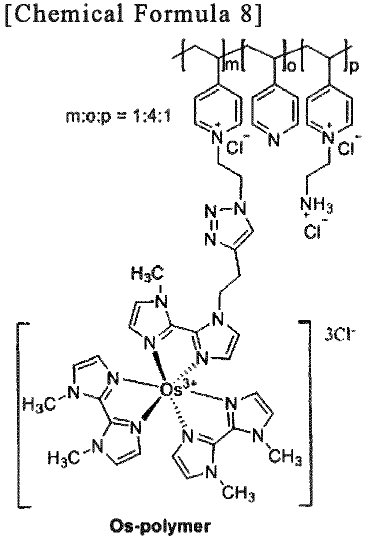

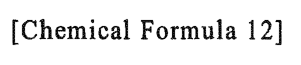

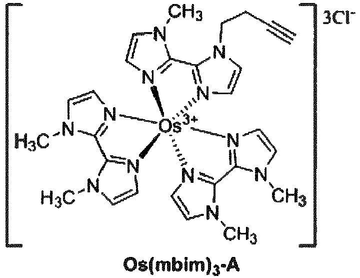

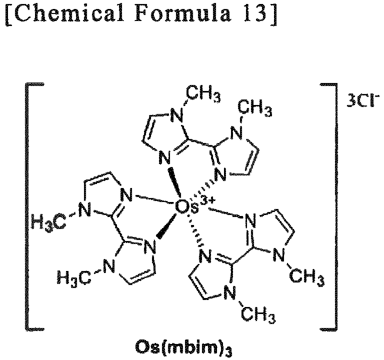

### Experimental materials/Conditions

Working electrode: glass carbon electrode (dia: 3.0 mm)
Reference electrode: Ag/AgCl electrode
Counter electrode: platinum rod

### Test parameters

- Equipment: EmStat (PalmSens Co.)
- Technique: cyclic voltammetry
- Potential range: -1.0 ~ 1.0 V
- Scan rate: 10 mV/s

**[Table 1]**

| **Compound** | ***E*_{pc} (V)** | ***Eₚₐ* (V)** |
|---|---|---|
| Os(mbim)₃-A | -0.150 | -0.263 |
| Os(mbim)₃ | -0.165 | -0.284 |
| Os-polymer | -0.160 | -0.290 |

As shown in FIG. 2 and Table 1, as a result of measuring the cyclic voltammetry curve of an oxidation-reduction polymer [Chemical Formula 8] together with osmium tris(III) (*N*,*N*'-dimethyl-2,2'-biimidazole) and osmium(III) (*N*,*N*'-dimethyl-2,2'-biimidazole)₂(N-butynyl-N'-methyl-2,2'-biimidazole), all three compounds showed an oxidation-reduction potential at approximately the same position. Therefore, it was indirectly confirmed that the performance of the oxidation-reduction polymer according to the present disclosure as an electron transport medium is the same as that of a single osmium complex.

## Claims

1. An oxidation-reduction polymer for an electron transport medium of an electrochemical biosensor, having any one structure of the following Chemical Formulas 1 to 4: 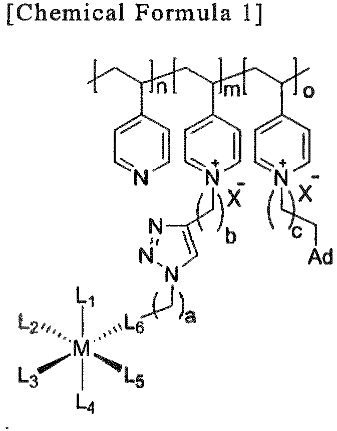 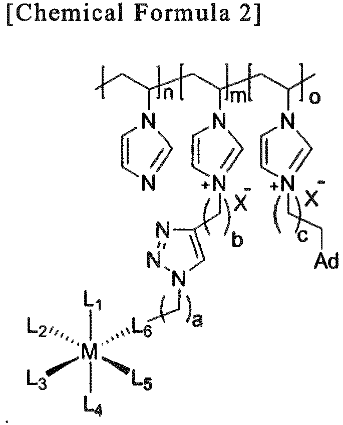 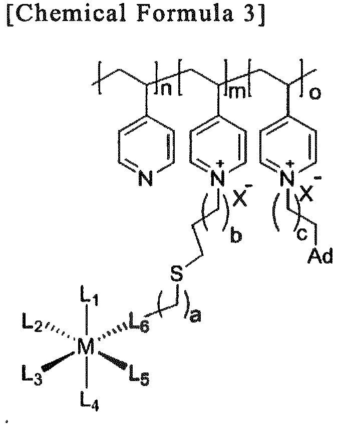 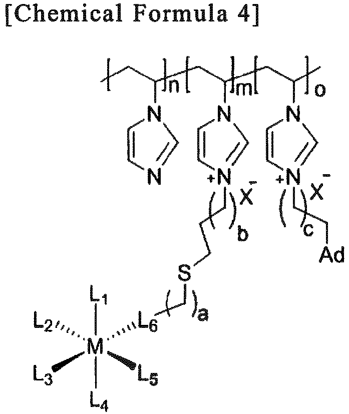 wherein,
M is a transition metal selected from the group consisting of Os, Rh, Ru, Ir, Fe and Co;
L₁ and L₂ are combined with each other to form a bidentate ligand selected from the following Chemical Formulas 5 to 7;
L₃ and L₄ are combined with each other to form a bidentate ligand selected from the following Chemical Formulas 5 to 7;
L₅ and L₆ are each combined with each other to form a bidentate ligand selected from the following Chemical Formulas 5 to 7; 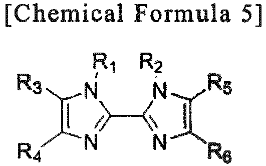 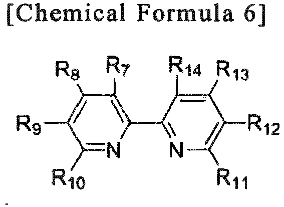 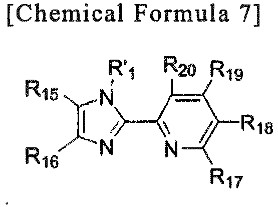
the R₁, R₂ and R'₁ are each independently selected from the group consisting of a substituted or unsubstituted alkyl group having 1 to 10 carbon atoms, a substituted or unsubstituted alcohol group having 1 to 20 carbon atoms, a substituted or unsubstituted alkylhalogen group having 1 to 20 carbon atoms, a substituted or unsubstituted thiol group having 1 to 20 carbon atoms, a substituted or unsubstituted alkyl azide group having 3 to 20 carbon atoms, a substituted or unsubstituted aryl azide group having 7 to 30 carbon atoms, a substituted or unsubstituted alkenyl group having 2 to 40 carbon atoms, a substituted or unsubstituted alkynyl group having 2 to 40 carbon atoms, a cyano group, a halogen group, deuterium and hydrogen,
the R₃ to R₂₀ are each independently selected from the group consisting of a substituted or unsubstituted alkyl group having 1 to 10 carbon atoms, a substituted or unsubstituted cycloalkyl group having 3 to 40 carbon atoms, a substituted or unsubstituted aryl group having 6 to 50 carbon atoms, a substituted or unsubstituted heteroaryl group having 3 to 50 carbon atoms, a substituted or unsubstituted alkoxy group having 1 to 20 carbon atoms, a substituted or unsubstituted alcohol group having 1 to 20 carbon atoms, a substituted or unsubstituted alkylhalogen group having 1 to 20 carbon atoms, a substituted or unsubstituted thiol group having 1 to 20 carbon atoms, a substituted or unsubstituted alkyl azide group having 3 to 20 carbon atoms, a substituted or unsubstituted aryl azide group having 7 to 30 carbon atoms, a substituted or unsubstituted aryloxy group having 6 to 30 carbon atoms, a substituted or unsubstituted alkylamino group having 1 to 20 carbon atoms, a substituted or unsubstituted arylamino group having 6 to 30 carbon atoms, a substituted or unsubstituted alkylsilyl group having 1 to 20 carbon atoms, a substituted or unsubstituted arylsilyl group having 6 to 30 carbon atoms, a substituted or unsubstituted arylalkylamino group having 1 to 50 carbon atoms, a substituted or unsubstituted alkenyl group having 2 to 40 carbon atoms, a substituted or unsubstituted alkynyl group having 2 to 40 carbon atoms, a cyano group, a halogen group, deuterium and hydrogen;
the Ad is selected from the group consisting of a primary and secondary amine group, an ammonium group, a halogen group, an epoxy group, an azide group, an acrylate group, an alkenyl group, an alkynyl group, a thiol group, isocyanate, an alcohol group, and a silane group;
the X is a counter ion;
the a is an integer of 1 to 15;
the b is an integer of 1 to 15;
the c is an integer of 1 to 15;
the m is an integer of 10 to 600;
the n is an integer of 10 to 600; and
the o is an integer of 0 to 600.

2. The oxidation-reduction polymer for an electron transport medium according to claim 1, wherein the compound of Chemical Formula 1 has a structure represented by the following Chemical Formula 8 or 9.

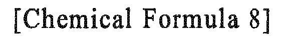

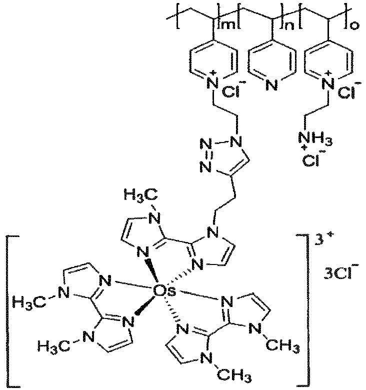

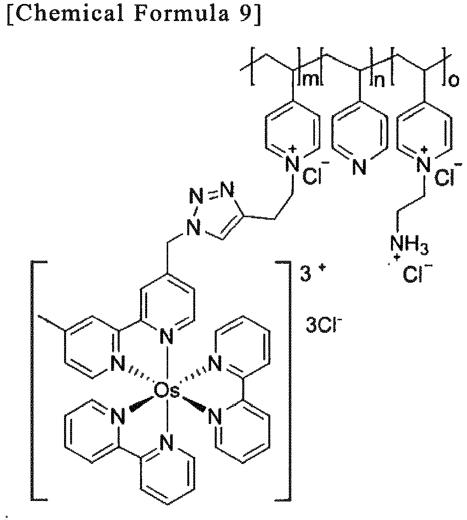

3. A method for preparing an oxidation-reduction polymer as set forth in claim 1, the method comprising the steps of:
(i) functionalizing polyvinylpyridine or polyimidazole to produce a polyvinylpyridine precursor or a polyimidazole precursor;
(ii) functionalizing the transition metal complex; and
(iii) reacting the polyvinylpyridine precursor or polyimidazole precursor produced in step (i) and the functionalized transition metal complex produced in step (ii) by a click reaction to prepare the oxidation-reduction polymer as set forth in claim 1.

4. The preparation method according to claim 3, wherein the click reaction of step (iii) is azide-alkyne Huisgen cycloaddition or thiol-ene reaction.

5. A composition for an electrochemical biosensor comprising:
an enzyme capable of subjecting a liquid biological sample to an oxidation-reduction reaction; and
the oxidation-reduction polymer as set forth in claim 1.

6. The composition according to claim 5, wherein the enzyme comprises:
at least one oxidoreductase selected from the group consisting of dehydrogenase, oxidase, and esterase; or
at least one oxidoreductase selected from the group consisting of dehydrogenase, oxidase, and esterase, and at least one cofactor selected from the group consisting of flavin adenine dinucleotide (FAD), nicotinamide adenine dinucleotide (NAD), and pyrroloquinoline quinone (PQQ).

7. The composition according to claim 5, wherein the enzyme is at least one selected from the group consisting of flavin adenine dinucleotide-glucose dehydrogenase (FAD-GDH) and nicotinamide adenine dinucleotide-glucose dehydrogenase.

8. The composition according to claim 5, further comprising one or more additives selected from the group consisting of surfactants, water-soluble polymers, and thickeners, and a crosslinking agent.

9. An electrochemical biosensor comprising the oxidation-reduction polymer according to claim 1.

10. The electrochemical biosensor according to claim 9, further comprising a sensing layer, a diffusion layer, a protection layer, two or more electrodes, an insulator and a substrate, comprising the oxidation-reduction polymer as set forth in claim 1.

11. The electrochemical biosensor according to claim 10, wherein the electrode is a 2-electrode consisting of an working electrode and a counter electrode, or a 3-electrode consisting of an working electrode, a counter electrode and a reference electrode.

12. The electrochemical biosensor according to claim 9, wherein the biological sample is blood.

## Patentansprüche

1. Oxidationsreduktionspolymer für ein Elektronentransportmedium eines elektrochemischen Biosensors mit einer beliebigen Struktur der folgenden chemischen Formeln 1 bis 4: 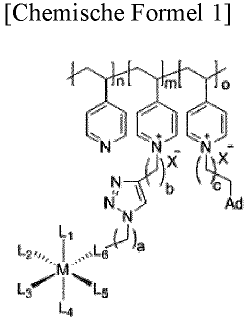 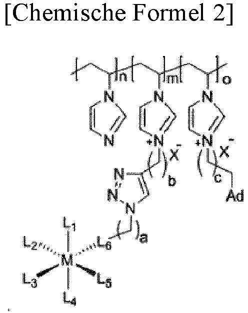 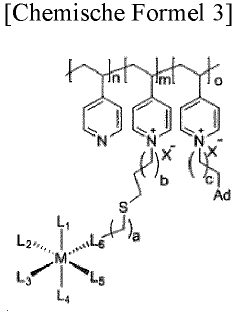 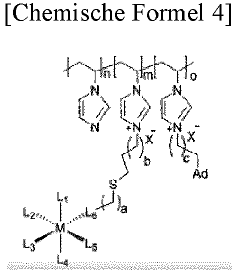 wobei
M ein Übergangsmetall ist, das aus der Gruppe bestehend aus Os, Rh, Ru, Ir, Fe und Co ausgewählt ist;
L₁ und L₂ miteinander kombiniert sind, um einen zweizahnigen Liganden zu bilden, der aus den folgenden chemischen Formeln 5 bis 7 ausgewählt ist;
L₃ und L₄ miteinander kombiniert sind, um einen zweizahnigen Liganden zu bilden, der aus den folgenden chemischen Formeln 5 bis 7 ausgewählt ist;
L₅ und L₆ jeweils miteinander kombiniert sind, um einen zweizahnigen Liganden zu bilden, der aus den folgenden chemischen Formeln 5 bis 7 ausgewählt wird; 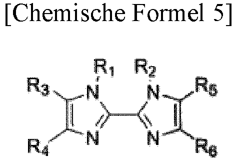 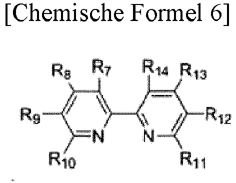 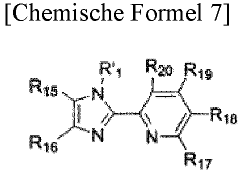
wobei R₁, R₂ und R'₁ jeweils unabhängig voneinander ausgewählt sind aus der Gruppe bestehend aus einer substituierten oder unsubstituierten Alkylgruppe mit 1 bis 10 Kohlenstoffatomen, einer substituierten oder unsubstituierten Alkoholgruppe mit 1 bis 20 Kohlenstoffatomen, einer substituierten oder unsubstituierten Alkylhalogengruppe mit 1 bis 20 Kohlenstoffatomen, einer substituierten oder unsubstituierten Thiolgruppe mit 1 bis 20 Kohlenstoffatomen, einer substituierten oder unsubstituierten Alkylazidgruppe mit 3 bis 20 Kohlenstoffatomen, einer substituierten oder unsubstituierten Arylazidgruppe mit 7 bis 30 Kohlenstoffatomen, einer substituierten oder unsubstituierten Alkenylgruppe mit 2 bis 40 Kohlenstoffatomen, einer substituierten oder unsubstituierten Alkinylgruppe mit 2 bis 40 Kohlenstoffatomen, einer Cyanogruppe, einer Halogengruppe, Deuterium und Wasserstoff,
wobei R₃ bis R₂₀ jeweils unabhängig voneinander ausgewählt sind aus der Gruppe bestehend aus einer substituierten oder unsubstituierten Alkylgruppe mit 1 bis 10 Kohlenstoffatomen, einer substituierten oder unsubstituierten Cycloalkylgruppe mit 3 bis 40 Kohlenstoffatomen, einer substituierten oder unsubstituierten Arylgruppe mit 6 bis 50 Kohlenstoffatomen, einer substituierten oder unsubstituierten Heteroarylgruppe mit 3 bis 50 Kohlenstoffatomen, einer substituierten oder unsubstituierten Alkoxygruppe mit 1 bis 20 Kohlenstoffatomen, einer substituierten oder unsubstituierten Alkoholgruppe mit 1 bis 20 Kohlenstoffatomen, einer substituierten oder unsubstituierten Alkylhalogengruppe mit 1 bis 20 Kohlenstoffatomen, einer substituierten oder unsubstituierten Thiolgruppe mit 1 bis 20 Kohlenstoffatomen, einer substituierten oder unsubstituierten Alkylazidgruppe mit 3 bis 20 Ko0 Kohlenstoffatomen, einer substituierten oder unsubstituierten Arylazidgruppe mit 7 bis 30 Kohlenstoffatomen, einer substituierten oder unsubstituierten Aryloxygruppe mit 6 bis 30 Kohlenstoffatomen, einer substituierten oder unsubstituierten Alkylaminogruppe mit 1 bis 20 Kohlenstoffatomen, einer substituierten oder unsubstituierten Arylaminogruppe mit 6 bis 30 Kohlenstoffatomen, einer substituierten oder unsubstituierten Alkylsilylgruppe mit 1 bis 20 Kohlenstoffatomen, einer substituierten oder unsubstituierten Arylsilylgruppe mit 6 bis 30 Kohlenstoffatomen, einer substituierten oder unsubstituierten Arylalkylaminogruppe mit 1 bis 50 Kohlenstoffatomen, einer substituierten oder unsubstituierten Alkenylgruppe mit 2 bis 40 Kohlenstoffatomen, einer substituierten oder unsubstituierten Alkinylgruppe mit 2 bis 40 Kohlenstoffatomen, einer Cyanogruppe, einer Halogengruppe, Deuterium und Wasserstoff;
wobei Ad ausgewählt ist aus der Gruppe bestehend aus einer primären und einer sekundären Amingruppe, einer Ammoniumgruppe, einer Halogengruppe, einer Epoxygruppe, einer Azidgruppe, einer Acrylatgruppe, einer Alkenylgruppe, einer Alkinylgruppe, einer Thiolgruppe, Isocyanat, einer Alkoholgruppe und einer Silangruppe;
wobei X ein Gegenion ist;
wobei a eine ganze Zahl von 1 bis 15 ist;
wobei b eine ganze Zahl von 1 bis 15 ist;
wobei c eine ganze Zahl von 1 bis 15 ist;
wobei m eine ganze Zahl von 10 bis 600 ist;
wobei n eine ganze Zahl von 10 bis 600 ist; und
wobei o eine ganze Zahl von 0 bis 600 ist.

2. Oxidationsreduktionspolymer für ein Elektronentransportmedium nach Anspruch 1, wobei die Verbindung der chemischen Formel 1 eine Struktur aufweist, die durch die folgende chemische Formel 8 oder 9 dargestellt wird.

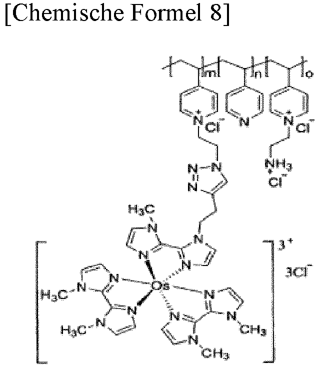

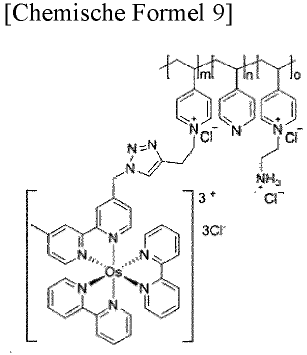

3. Verfahren zur Vorbereitung eines Oxidationsreduktionspolymers nach Anspruch 1, wobei das Verfahren die folgenden Schritte umfasst:
(i) Funktionalisieren von Polyvinylpyridin oder Polyimidazol, um einen Polyvinylpyridin-Vorläufer oder einen Polyimidazol-Vorläufer herzustellen;
(ii) Funktionalisieren des Übergangsmetallkomplexes; und
(iii) Umsetzen des in Schritt (i) hergestellten Polyvinylpyridin-Vorläufers oder Polyimidazol-Vorläufers und des in Schritt (ii) hergestellten funktionalisierten Übergangsmetallkomplexes durch eine Klick-Reaktion, um das Oxidationsreduktionspolymer nach Anspruch 1 vorzubereiten.

4. Vorbereitungsverfahren nach Anspruch 3, wobei es sich bei der Klick-Reaktion von Schritt (iii) um eine Azid-Alkin-Huisgen-Cycloaddition oder eine Thiol-En-Reaktion handelt.

5. Zusammensetzung für einen elektrochemischen Biosensor, die Folgendes umfasst:
ein Enzym, das eine flüssige biologische Probe einer Oxidationsreduktionsreaktion unterziehen kann; und
das Oxidationsreduktionspolymer nach Anspruch 1.

6. Zusammensetzung nach Anspruch 5, wobei das Enzym Folgendes umfasst:
mindestens eine Oxidoreduktase, die aus der Gruppe bestehend aus Dehydrogenase, Oxidase und Esterase ausgewählt ist; oder
mindestens eine Oxidoreduktase, die aus der Gruppe bestehend aus Dehydrogenase, Oxidase und Esterase ausgewählt ist, und mindestens einen Cofaktor, der aus der Gruppe bestehend aus Flavinadenindinukleotid (FAD), Nicotinamidadenindinukleotid (NAD) und Pyrrolochinolinchinon (PQQ) ausgewählt ist.

7. Zusammensetzung nach Anspruch 5, wobei es sich bei dem Enzym um mindestens eines handelt, das aus der Gruppe bestehend aus Flavinadenindinukleotid-Glukosedehydrogenase (FAD-GDH) und Nicotinamidadenindinukleotid-Glukosedehydrogenase ausgewählt ist.

8. Zusammensetzung nach Anspruch 5, die außerdem einen oder mehrere Zusatzstoffe, der bzw. die aus der Gruppe bestehend aus Tensiden, wasserlöslichen Polymeren und Verdickungsmitteln ausgewählt ist bzw. sind, und ein Vernetzungsmittel umfasst.

9. Elektrochemischer Biosensor, der das Oxidationsreduktionspolymer nach Anspruch 1 umfasst.

10. Elektrochemischer Biosensor nach Anspruch 9, der außerdem eine Sensorschicht, eine Diffusionsschicht, eine Schutzschicht, zwei oder mehr Elektroden, einen Isolator und ein Substrat umfasst, umfassend das Oxidationsreduktionspolymer nach Anspruch 1.

11. Elektrochemischer Biosensor nach Anspruch 10, wobei es sich bei der Elektrode um eine 2-Elektrode bestehend aus einer Arbeitselektrode und einer Gegenelektrode oder um eine 3-Elektrode bestehend aus einer Arbeitselektrode, einer Gegenelektrode und einer Referenzelektrode handelt.

12. Elektrochemischer Biosensor nach Anspruch 9, wobei es sich bei der biologischen Probe um Blut handelt.

## Revendications

1. Polymère d'oxydo-réduction destiné à un milieu de transport d'électrons d'un biocapteur électrochimique, présentant l'une quelconque des structures des formules chimiques 1 à 4 suivantes : 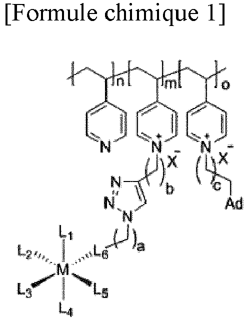 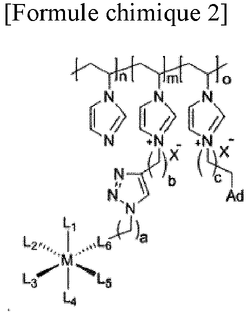 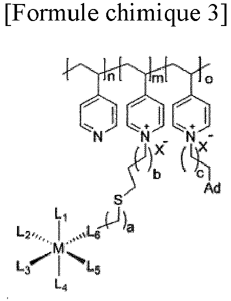 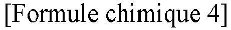 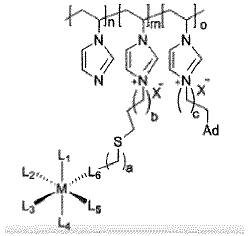 dans lesquelles
M est un métal de transition choisi dans le groupe constitué par Os, Rh, Ru, Ir, Fe et Co ;
L₁ et L₂ sont combinés l'un avec l'autre pour former un ligand bidenté choisi parmi les formules chimiques 5 à 7 suivantes ;
L₃ et L₄ sont combinés l'un avec l'autre pour former un ligand bidenté choisi parmi les formules chimiques 5 à 7 suivantes ;
L₅ et L₆ sont chacun combinés l'un avec l'autre pour former un ligand bidenté choisi parmi les formules chimiques 5 à 7 suivantes ; 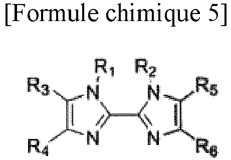 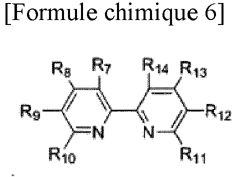 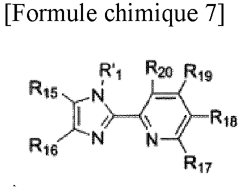
les radicaux R₁, R₂ et R'₁ sont chacun indépendamment choisis dans le groupe constitué par un groupe alkyle substitué ou non substitué ayant 1 à 10 atomes de carbone, un groupe alcool substitué ou non substitué ayant 1 à 20 atomes de carbone, un groupe alkylhalogéno substitué ou non substitué ayant 1 à 20 atomes de carbone, un groupe thiol substitué ou non substitué ayant 1 à 20 atomes de carbone, un groupe azoture d'alkyle substitué ou non substitué ayant 3 à 20 atomes de carbone, un groupe azoture d'aryle substitué ou non substitué ayant 7 à 30 atomes de carbone, un groupe alcényle substitué ou non substitué ayant 2 à 40 atomes de carbone, un groupe alcynyle substitué ou non substitué ayant 2 à 40 atomes de carbone, un groupe cyano, un groupe halogéno, le deutérium et l'hydrogène,
les radicaux R₃ à R₂₀ sont chacun indépendamment choisis dans le groupe constitué par un groupe alkyle substitué ou non substitué ayant 1 à 10 atomes de carbone, un groupe cycloalkyle substitué ou non substitué ayant 3 à 40 atomes de carbone, un groupe aryle substitué ou non substitué ayant 6 à 50 atomes de carbone, un groupe hétéroaryle substitué ou non substitué ayant 3 à 50 atomes de carbone, un groupe alcoxy substitué ou non substitué ayant 1 à 20 atomes de carbone, un groupe alcool substitué ou non substitué ayant 1 à 20 atomes de carbone, un groupe alkylhalogéno substitué ou non substitué ayant 1 à 20 atomes de carbone, un groupe thiol substitué ou non substitué ayant 1 à 20 atomes de carbone, un groupe azoture d'alkyle substitué ou non substitué ayant 3 à 20 atomes de carbone, un groupe azoture d'aryle substitué ou non substitué ayant 7 à 30 atomes de carbone, un groupe aryloxy substitué ou non substitué ayant 6 à 30 atomes de carbone, un groupe alkylamino substitué ou non substitué ayant 1 à 20 atomes de carbone, un groupe arylamino substitué ou non substitué ayant 6 à 30 atomes de carbone, un groupe alkylsilyle substitué ou non substitué ayant 1 à 20 atomes de carbone, un groupe arylsilyle substitué ou non substitué ayant 6 à 30 atomes de carbone, un groupe arylalkylamino substitué ou non substitué ayant 1 à 50 atomes de carbone, un groupe alcényle substitué ou non substitué ayant 2 à 40 atomes de carbone, un groupe alcynyle substitué ou non substitué ayant 2 à 40 atomes de carbone, un groupe cyano, un groupe halogéno, le deutérium et l'hydrogène ;
Ad est choisi dans le groupe constitué par un groupe amine primaire et secondaire, un groupe ammonium, un groupe halogéno, un groupe époxy, un groupe azoture, un groupe acrylate, un groupe alcényle, un groupe alcynyle, un groupe thiol, un isocyanate, un groupe alcool et un groupe silane ;
X est un contre-ion ;
a est un nombre entier de 1 à 15 ;
b est un nombre entier de 1 à 15 ;
c est un nombre entier de 1 à 15 ;
m est un nombre entier de 10 à 600 ;
n est un nombre entier de 10 à 600 ; et
o est un entier de 0 à 600.

2. Polymère d'oxydo-réduction destiné à un milieu de transport d'électrons selon la revendication 1, dans lequel le composé de formule chimique 1 a une structure représentée par la formule chimique 8 ou 9 suivante.

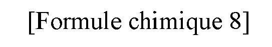

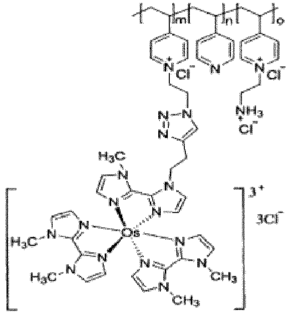

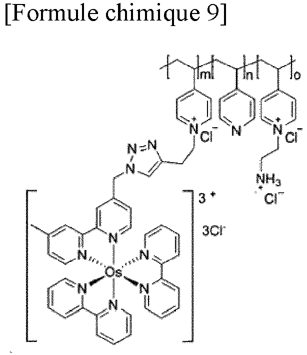

3. Procédé de préparation d'un polymère d'oxydo-réduction selon la revendication 1, le procédé comprenant les étapes consistant à :
(i) fonctionnaliser de la polyvinylpyridine ou du polyimidazole pour produire un précurseur de polyvinylpyridine ou un précurseur de polyimidazole ;
(ii) fonctionnaliser le complexe de métal de transition ; et
(iii) faire réagir le précurseur de polyvinylpyridine ou le précurseur de polyimidazole produit à l'étape (i) et le complexe de métal de transition fonctionnalisé produit à l'étape (ii) par une réaction clic pour préparer le polymère d'oxydo-réduction selon la revendication 1.

4. Procédé de préparation selon la revendication 3, dans lequel la réaction clic de l'étape (iii) est une cycloaddition azoture-alcyne de Huisgen ou une réaction thiol-ène.

5. Composition pour biocapteur électrochimique comprenant :
une enzyme capable de soumettre un échantillon biologique liquide à une réaction d'oxydoréduction, et
le polymère d'oxydo-réduction selon la revendication 1.

6. Composition selon la revendication 5, dans laquelle l'enzyme comprend :
au moins une oxydoréductase choisie dans le groupe constitué par la déshydrogénase, l'oxydase et l'estérase, ou
au moins une oxydoréductase choisie dans le groupe constitué par la déshydrogénase, l'oxydase et l'estérase, et au moins un cofacteur choisi dans le groupe constitué par la flavine adénine dinucléotide (FAD), la nicotinamide adénine dinucléotide (NAD) et la pyrroloquinoléine quinone (PQQ).

7. Composition selon la revendication 5, dans laquelle l'enzyme est au moins une enzyme choisie dans le groupe constitué par la flavine adénine dinucléotide-glucose déshydrogénase (FAD-GDH) et la nicotinamide adénine dinucléotide-glucose déshydrogénase.

8. Composition selon la revendication 5, comprenant en outre un ou plusieurs additifs choisis dans le groupe constitué par les tensioactifs, les polymères hydrosolubles et les épaississants, et un agent de réticulation.

9. Biocapteur électrochimique comprenant le polymère d'oxydo-réduction selon la revendication 1.

10. Biocapteur électrochimique selon la revendication 9, comprenant en outre une couche de détection, une couche de diffusion, une couche de protection, deux électrodes ou plus, un isolant et un substrat, comprenant le polymère d'oxydo-réduction selon la revendication 1.

11. Biocapteur électrochimique selon la revendication 10, dans lequel l'électrode est une électrode double constituée d'une électrode de travail et d'une contre-électrode, ou une électrode triple constituée d'une électrode de travail, d'une contre-électrode et d'une électrode de référence.

12. Biocapteur électrochimique selon la revendication 9, dans lequel l'échantillon biologique est du sang.
